# EUROPEAN PATENT SPECIFICATION

(11) **EP 4 349 521 B1**
(45) Date of publication and mention of the grant of the patent: **22.04.2026**
(21) Application number: 22199806.5
(22) Date of filing: 05.10.2022
(51) Int. Cl.: B23K 26/08, A61F 2/91, B23K 26/36, B23K 26/38, B23K 37/02, B23K 37/04, B23K 37/053, B23K 37/0533, B23K 37/0538, B23K 101/06

(54) **PRODUCTION SYSTEM FOR PRODUCING A MEDICAL DEVICE FROM A TUBE**
FERTIGUNGSSYSTEM ZUR HERSTELLUNG EINES MEDIZINPRODUKTS AUS EINEM ROHR
SYSTÈME DE PRODUCTION POUR PRODUIRE UN DISPOSITIF MÉDICAL À PARTIR D'UN TUBE

(43) Date of publication of application: 10.04.2024
(73) Proprietor: BIOTRONIK AG, 8180 Bülach (CH)
(72) Inventor: Klemm, Ralf, 18209 Parkentin (DE); Neckin, André, 18059 Rostock (DE); Kühn, Johannes, 18057 Rostock (DE); Müller, Jens, 18106 Rostock (DE)
(74) Representative: WSL Patentanwälte Partnerschaft mbB

(56) References cited:
- CN-A- 113 857 766
- CN-A- 113 977 116
- CN-B- 111 730 282
- DE-A1- 102008 011 232

## Description

The instant invention concerns a production system for producing a medical device from a tube. The instant invention in particular concerns a production system for producing a stent or a scaffold from a tube, such as a tube made from a nitinol material, a steel material, a cobalt-chrome (Co-Cr) material, or a bioresorbable magnesium material or any other suitable material.

In a production system of this kind a cylindrical tube is held by means of a positioning device such that a portion of the tube extends from the positioning device along a longitudinal direction. The guiding device serves to guide the portion of the tube, and a laser cutting or micromachining device is used to form or process the medical device from the portion of the tube.

Using the laser cutting device for example a stent or scaffold structure is formed on the tube by cutting openings or other shapes into the tube. Whereas the positioning device is used to move the tube longitudinally along the longitudinal direction or rotationally about the rotational direction in order to form a desired shape on the tube to obtain a medical device, the guiding device is designed to guide the portion of the tube extending from the positioning device in such a way that the portion of the tube is maintained in a specified position in a plane perpendicular to the longitudinal direction at the location of the laser cutting device. The guiding device herein shall allow that a distance between the laser cutting device and the tube is maintained at a specified value, such that a laser beam produced by the laser cutting device may be focused on the tube in order to cut the tube with a laser cut line having a defined width and hence allowing a specified radius of curvature of the cut line.

In conventional production systems of this kind, a guiding device is used which utilizes a guide element made from a PTFE (polytetrafluorethylene) material. The guide element herein comprises a groove in which the tube is received and guided with small friction such that the tube is supported in order to maintain a proper positioning of tube for interaction with the laser cutting device.

The use of a PTFE material allows for a low friction, however comes with the drawback of a significant cold flow behavior, caused by strain and pressure on the material, and a wear and tear caused by movement of the tube with respect to the guide element. A cold flow behavior as well as a wear and tear may have an influence on the guiding characteristics of the guide element, such that possibly the accuracy of the positioning of the tube with respect to the laser cutting device can no longer be maintained.

In order to counteract influences of a cold flow behavior and a wear and tear, a guiding surface of the guide element made from a PTFE material may be made large. However, this has the disadvantage that a length of the tube which cannot be used for forming medical devices is increased, as a substantial length is received on the guiding device and cannot be accessed by the laser cutting device.

In addition, guiding devices as currently known require - in addition to a vertical and horizontal alignment - an angular adjustability in order to provide for an alignment also of the angular position. This impacts costs and complexity of the production, as the alignment procedure is cumbersome.

DE 10 2008 011 232 A1, on which the preamble of claim 1 is based, relates to a device for laser cutting medical stents from tubular blanks, with a laser cutting head and a manipulating device which is configured to move the blank translationally in the direction of its longitudinal axis and about its longitudinal axis in rotation. A tube guide arrangement is arranged in a fixed position on the machine.

It is an object of the instant invention to provide a production system for producing a medical device from a tube which allows for an easy and cost-efficient, yet reliable and precise production of a medical device, in particular in the shape of a stent or a scaffold.

This object is achieved by means of a production system comprising the features of claim 1.

Accordingly, in one aspect, a production system for producing a medical device from a tube comprises: a positioning device for positioning the tube, the positioning device being configured to hold the tube such that a portion of the tube extends from the positioning device along a longitudinal direction; a guiding device for guiding said portion of the tube; a laser cutting device for forming said medical device from said portion of the tube; wherein said guiding device comprises a multiplicity of bearing elements each configured to abut said portion of the tube and each having a convex curvature in a plane spanned by the longitudinal direction and a transverse direction perpendicular to the longitudinal direction. Further, the guiding device comprises a first guiding section having a first body and a second guiding section having a second body. A first subgroup of the multiplicity of bearing elements is arranged on the first body of the first guiding section, and a second subgroup of the multiplicity of bearing elements is arranged on the second body of the second guiding section. The first subgroup may comprise one or multiple bearing elements. The second subgroup may comprise the remainder of the bearing elements which are not part of the first subgroup. The first subgroup of the multiplicity of bearing elements is rotationally fixed to the first body of the first guiding section. In addition or alternatively, the second subgroup of the multiplicity of bearing elements is rotationally fixed to the second body of the second guiding section. The bearing elements hence are fixed to the associated guiding section such that the bearing elements do not move during operation of the production system, but are held rotationally fixed on the associated guiding section. By fixing the bearings elements to the guiding section, a play within the guiding device may be reduced to a minimum, as tolerances which come with movable elements are avoided.

The production system comprises a positioning device for positioning the tube, the positioning device being configured to hold the tube such that a portion of the tube extends from the positioning device along a longitudinal direction. The positioning device in particular may comprise a fixation device, such as a clamping chuck, for holding the tube such that the tube extends from the positioning device towards the laser cutting device. In addition, the positioning device may comprise an adjustment apparatus allowing to move the tube along the longitudinal direction and/or along a rotational direction about the longitudinal direction in order to move the tube with respect to the laser cutting device such that a medical device, such as a stent or a scaffold, may be formed on the tube using the laser cutting device. Using the laser cutting device, a suitable structure is cut into the (cylindrical) tube and, once the forming is complete, the medical device is separated from the tube.

The positioning device in particular may comprise an electric motor configured to move the clamping chuck longitudinally along the longitudinal direction and/or rotationally about the longitudinal direction.

The guiding device serves to guide the portion of the tube extending from the positioning device such that the tube is positioned with respect to the laser cutting device for interacting with a laser beam produced by the laser cutting device. The guiding device herein comprises a multiplicity of bearing elements, each bearing element having a curved shape for bearing the portion of the tube and hence for providing a support for the tube in order to properly position the tube for interaction with the laser cutting device. In particular, the bearing elements comprise a convex curvature in a plane spanned by the longitudinal direction and a transverse (radial) direction perpendicular to the longitudinal direction. By means of the convex curvature the bearing elements are designed to abut the tube at a spatially confined, spot-like location, thus reducing the size of a surface area in which a contact between the bearing elements and the tube is established for guiding the tube.

By reducing the size of the contact area, frictional forces in between the bearing elements and the tube during a cutting process are decreased. Beneficially, each bearing element abuts the tube only at a point location having a finite spot size due to the contact action between the bearing element and the tube (causing the bearing element to dig into - on a microscopic scale - the tube when being in contact with the tube).

Because the bearing elements each are curved in a plane spanned by the longitudinal direction and a direction transverse (radial) to the longitudinal direction, each bearing element provides for a support of the tube within the plane spanned by the longitudinal direction and the direction transverse to the longitudinal direction. Hence, the bearing elements in combination may provide no or only a limited angular support (in particular if the bearing elements are arranged in a common plane), such that the tube is positioned by the guiding device in a vertical direction and a transverse, horizontal direction, but not in its angular position. This facilitates the alignment of the guiding device with respect to the positioning device, in particular in that no angular alignment is required.

In one embodiment, the bearing elements are made from a material comprising a hardness larger than the hardness of the material of the tube.

In particular, the bearing elements may be made from a rolling bearing steel material, a ceramics material or a hard metal material. In turn, the tube may for example be made from a nitinol material, a stainless steel material, a cobalt-chrome (Co-Cr) material, or a bioresorbable magnesium material.

Commonly so far, a plastic material such as PTFE material is used for guiding in a guiding device, the PTFE material having a hardness smaller than the tube. As hence the tube is harder than the guiding material, the surface roughness of the tube - in conventional systems -predominantly determines the friction in between the tube and the guiding device. Whereas conventionally the material of the guiding device is less hard than the material of the tube, it is proposed herein to reverse this relation in that a material is used for the bearing elements which is harder than the material of the tube. By using bearing elements having a small surface roughness and by reducing a contact area in between the bearing elements and the tube, a beneficial guiding with reduced frictional forces and a reduced wear and tear can be achieved, allowing hence for a guiding device having an increased operational span of life.

Rolling bearing steel (e.g. 100Cr6, 100Cr2, 85Cr2, 105Cr4, 100CrMnSi6-3 or 100CrMo7) may for example be heat treated (e.g. martensitic-hardened and tempered at low temperatures), which may provide for a consistent high hardness, for example in between 55 HRC to 70 HRC, e.g. between 60 and 65 HRC, distributed over the circumference and cross-section.

By using a material such as a rolling bearing steel material, a ceramics material or a hard metal material, in addition bearing elements with a small surface roughness may be obtained, hence reducing friction between the bearing elements and the tube.

In one embodiment, at least one of the bearing elements may be formed by a spherical ball. In another embodiment, at least one of the bearing elements may be formed by a cylindrical pin. For example, all bearing elements may be formed by spherical balls or by cylindrical pins. In other embodiments, at least one of the bearing elements is a spherical ball, whereas the other bearing elements are cylindrical pins. In yet other embodiments, at least one of the bearing elements is a cylindrical pin, whereas the other bearing elements are formed by spherical balls.

By using bearing elements in the shape of spherical balls or cylindrical pins, standard bearing elements may be used, which may be obtained cost efficiently, hence reducing the overall costs of the production system.

In one embodiment, the guiding device comprises three bearing elements. In another embodiment, the guiding device comprises four bearing elements. In yet other embodiments, the guiding device comprises less than three bearing elements or more than four bearing elements.

In one embodiment, the guiding device comprises four bearing elements in the shape of spherical balls.

In another embodiment, the guiding device comprises three bearing elements in the shape of cylindrical pins.

In one embodiment, at least one bearing element formed by a cylindrical pin extends longitudinally along an axis of extension, wherein the axis of extension lies in a plane oriented perpendicularly to the longitudinal direction. Accordingly, the bearing element in the shape of the cylindrical pin extends transversely with respect to the longitudinal direction, such that the bearing element is convexly curved in a plane spanned by the longitudinal direction and a transverse (radial) direction with respect to the longitudinal direction. Because the (cylindrical) tube extends along the longitudinal direction and the bearing element extends along the axis of extension transverse to the longitudinal direction, the bearing element and the tube come into contact at a confined, spot-like location, such that the contact area in between the tube and the bearing element is limited in size.

Beneficially, all bearing elements having the shape of cylindrical pins extend longitudinally along an associated axis of extension transversely to the longitudinal direction and hence in an associated plane oriented perpendicularly to the longitudinal direction.

In one embodiment, the bearing elements are arranged in a common plane oriented perpendicularly to the longitudinal direction.

The bearing elements herein beneficially have the same diameter, such that equal bearing elements are distributed within the common plane.

If the bearing elements are formed as cylindrical pins, this is to be understood in that the axes of extension of the different bearing elements lie in a common plane.

If the bearing elements are arranged in a common plane, the guiding device provides no or a very limited angular support for the tube. The guiding device hence provides for a support and guidance for the tube primarily in the common plane, which is oriented perpendicularly to the longitudinal direction. This may facilitate production in that no angular alignment of the guiding device with respect to the positioning device is required, hence easing the setup of the production procedure when using the production system for producing medical devices.

In another embodiment, at least one of the bearing elements is arranged in a first plane oriented perpendicularly to the longitudinal direction, and at least one other of the bearing elements is arranged in a second plane oriented perpendicularly to the longitudinal direction and displaced with respect to said first plane along the longitudinal direction. Different bearing elements hence are arranged in different planes. For example, a first group of bearing elements may be arranged in a first plane, whereas a second group of bearing elements may be arranged in a second plane. In this way, it may be achieved that the guiding device in addition to a support along a vertical direction and a transverse, horizontal direction also provides for an angular support.

Hence, one or multiple bearing elements are arranged on the first guiding section, and one or multiple bearing elements are arranged on the second guiding section. For example, a first pair of bearing elements in the shape of spherical balls may be arranged on the first guiding section, and a second pair of bearing elements in the shape of spherical balls may be arranged on the second guiding section. In another embodiment, a pair of bearing elements in the shape of cylindrical pins may be arranged on the first guiding section, and a single bearing element in the shape of a cylindrical pin may be arranged on the second guiding section. In other embodiments, other combinations of bearing elements may be used.

In one embodiment, the first guiding section and the second guiding section are configured to receive the portion of the tube therebetween, such that the tube is guided, in an operational state, in between the first guiding section and the second guiding section.

The first guiding section and the second guiding section herein may be tensioned with respect to one another to apply a guiding pressure to the portion of the tube received between the first guiding section and the second guiding section, such that the bearing elements are forced to contact the tube with a predefined contact pressure in order to achieve a reliable, precise guiding of the tube in between the guiding sections.

A tensioning device for tensioning the first guiding section with respect to the second guiding section may for example be formed by a mechanical spring or by a hydraulic or pneumatic tensioning device for exerting a defined tensioning force in between the guiding sections of the guiding device.

In one embodiment, the first body comprises at least one first receptacle in which one of the bearing elements of said first subgroup is received. In addition or alternatively, the second body comprises at least one second receptacle in which one of the bearing elements of the second subgroup is received. Each bearing element hence may be received in a corresponding receptacle of the associated body, such that the bearing elements are held fixed on the guiding sections for interacting with the tube received in between the guiding sections.

In one embodiment, the bearing elements are clamped in the respective receptacle using for example a fixation element such as a clamping screw. By clamping the bearing elements in the receptacles, the bearing elements are held in place and are rotationally fixed with respect to the body of the associated guiding section, such that the bearing elements do not move, in particular do not rotate when moving the tube with respect to the guiding device during operation of the production system.

In one embodiment, the laser cutting device is configured to form the medical device on an end of the portion of the tube extending longitudinally from the positioning device. The guiding device herein is arranged, when viewed along the longitudinal direction, in between the positioning device and the laser cutting device. The laser cutting device hence is configured to form the medical device from a free end of the tube, wherein the guiding device provides for a support and guidance of the portion of the tube extending from the positioning device such that the end of the tube on which the medical device is formed using the laser cutting device is positioned accurately with respect to the laser cutting device to achieve a precise cutting action.

Using bearing elements as proposed herein, a guiding device may be provided which has a reduced length, for example a length smaller than 15 mm or even smaller than 10 mm (measured along the longitudinal direction). This allows to reduce a length of the tube which is not usable for forming medical devices.

By using bearing elements which may be obtained as standard bearing elements, for example in the shape of standard bearing balls or bearing pins, the cost of the guiding device and hence the overall cost of the production system may be reduced. Because the bearing elements are formed by a hard material, the operational lifespan of the guiding device may be increased by reducing a wear and tear of the guiding device, further reducing the costs for the production system and in addition reducing maintenance requirements of the production system.

The idea of the invention shall subsequently be described in more detail with reference to the embodiments shown in the figures. Herein:
- Fig. 1: shows a schematic drawing of a production system for producing a medical device using a laser cutting device from a tube;
- Fig. 2: shows an embodiment of a guiding device for guiding the tube in between a positioning device and a laser cutting device;
- Fig. 3: shows a front view of the arrangement of Fig. 2;
- Fig. 4: shows a view of bearing elements of the guiding device;
- Fig. 5: shows another view of the bearing elements of the guiding device:
- Fig. 6: shows another perspective view of the guiding device;
- Fig. 7: shows a cross-sectional view of the guiding device, along a plane perpendicular to a longitudinal direction along which a tube extends;
- Fig. 8: shows another cross-sectional view of the guiding device, along a plane spanned by the longitudinal direction and a vertical direction;
- Fig. 9: shows yet another cross-sectional view of the guiding device, along a plane spanned by the longitudinal direction and a transverse, horizontal direction;
- Fig. 10: shows a view of another embodiment of a guiding device:
- Fig. 11: shows another view of the guiding device of Fig. 10;
- Fig. 12: shows a cross-sectional view of the guiding device, along a plane perpendicular to the longitudinal direction along which a tube extends;
- Fig. 13: shows another cross-sectional view of the guiding device, along a plane spanned by the longitudinal direction and a vertical direction; and
- Fig. 14: shows yet another cross-sectional view of the guiding device, along a plane spanned by the longitudinal direction and a transverse, horizontal direction.

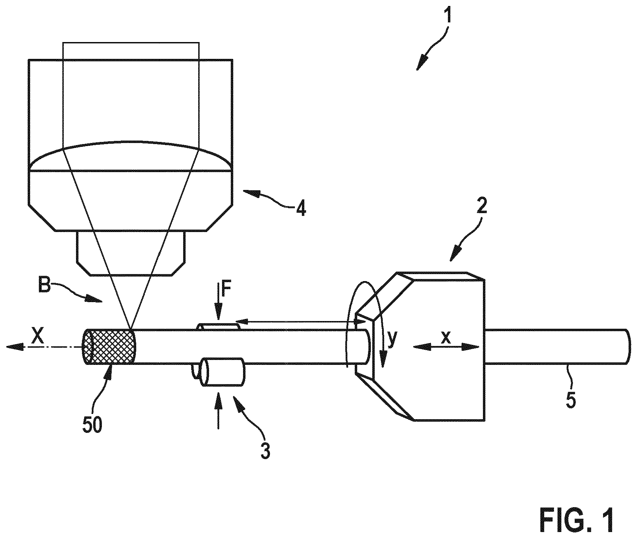

Fig. 1 shows a schematic view of a production system 1 which is used to produce medical devices 50 in the shape of e.g. stents of scaffolds from a tube 5 made for example of a nitinol material, a stainless steel material, or a bioresorbable material such as a magnesium material.

The production system 1 comprises a positioning device 2 which serves to hold the tube 5 using e.g. a clamping chuck and to position the tube 5 with respect to a laser cutting device 4. The positioning device 2 may allow for a translational movement of the tube 5 along a longitudinal direction X and a rotational movement of the tube 5 along a rotational direction Y about the longitudinal direction X, such that the tube 5 may be moved with respect to the laser cutting device 4 in order to control an interaction of the tube 5 with the laser cutting device 4 in a spatially defined manner to form a structure on the tube 5 for example by cutting openings or other shapes into the tube 5.

As it is visible from Fig. 1, the positioning device 2 is configured to hold the tube 5 such that a portion of the tube 5 longitudinally extends from the positioning device 2 in the direction of the laser cutting device 4. The laser cutting device 4 herein is positioned with respect to the tube 5 such that a medical device 50 may be formed on a free end of the tube 5, a laser beam B being directed towards the tube 5 and being focused such that a cutting location (approximately) coincides with a focal point of the laser beam B on the tube 5.

For achieving a precise and controlled cutting action of the laser cutting device 4 on the tube 5, it is crucial that a distance of the laser cutting device 4 with respect to the tube 5 is established and maintained in a precise manner. For this, a guiding device 3 is arranged in between the positioning device 2 and the laser cutting device 4 (when viewed along the longitudinal direction X), the guiding device 3 serving to guide the tube 5 and to provide for a support of the tube 5 such that the tube 5 is positioned with respect to the laser cutting device 4 in a stable manner.

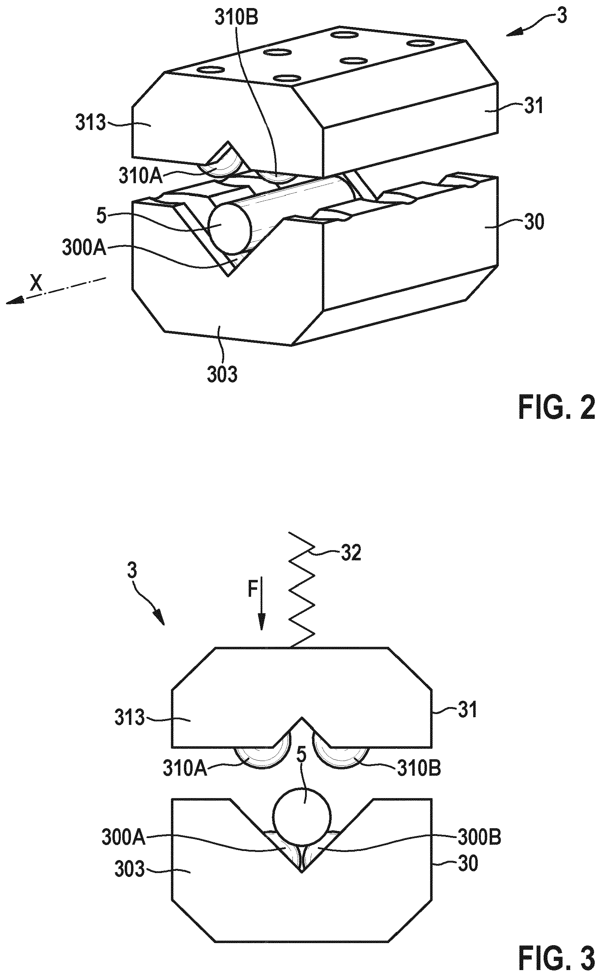

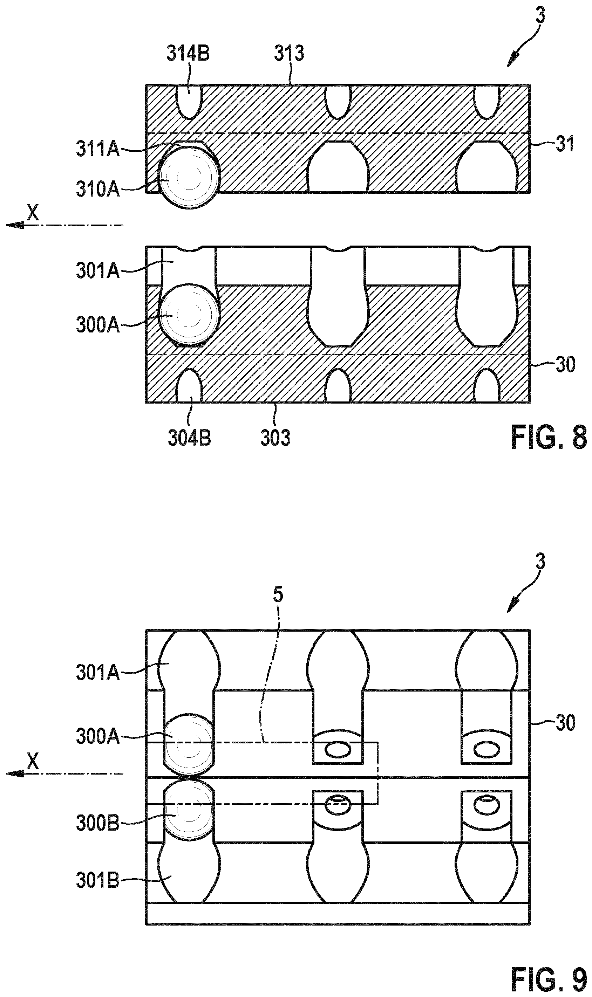

Referring now to Figs. 2 to 9, in one embodiment the guiding device 3 comprises guiding sections 30, 31, in between which a tube 5 is received for guiding the tube 5 in between the positioning device 2 and the laser cutting device 4, as shown in Fig. 1. Each guiding section 30, 31 comprises a body 303, 313, the body 303, 313 forming a groove in which the tube 5 is received.

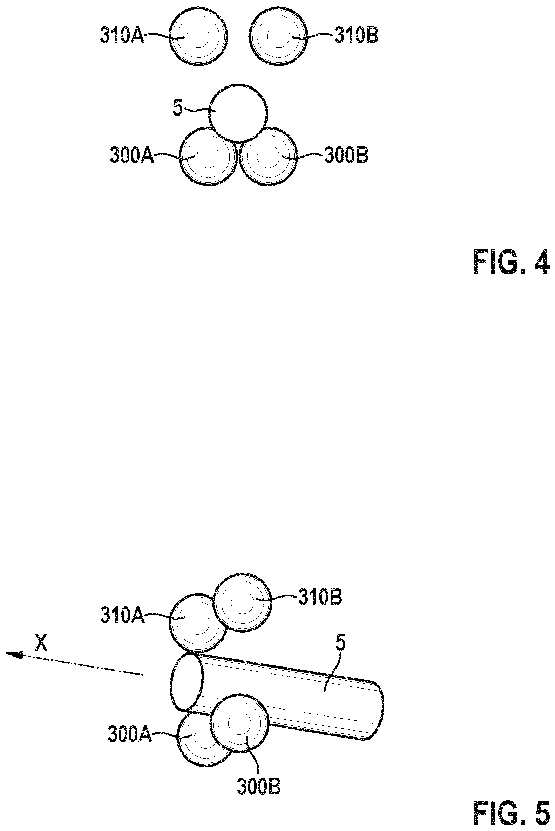

As visible from Figs. 2 and 3 in view of Figs. 4 and 5, each guiding section 30, 31 comprises, in the shown embodiment, a pair of bearing elements 300A, 300B, 310A, 310B formed by spherical balls.

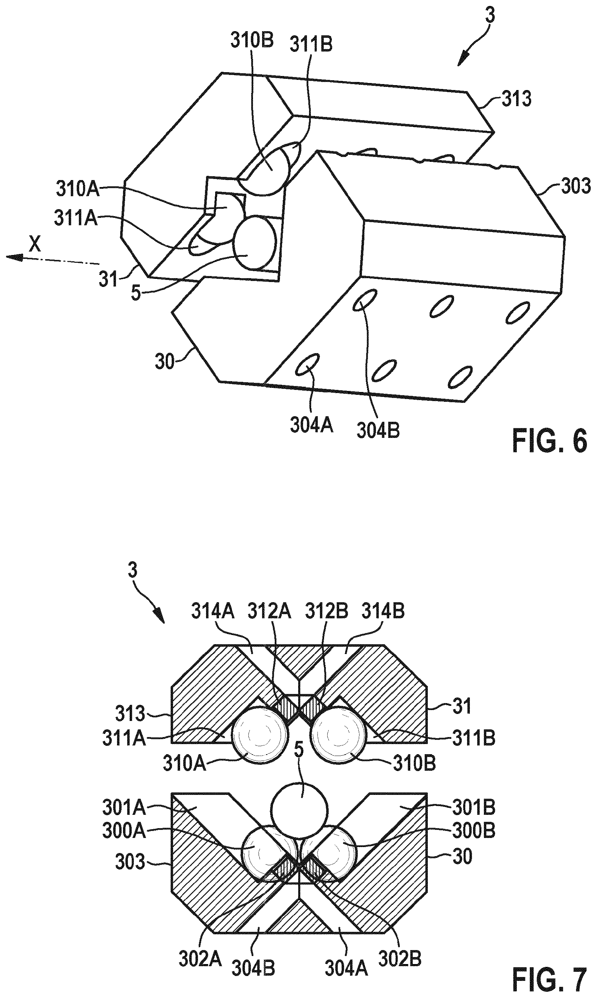

As further visible from the cross-sectional views of Figs. 7 to 9, the bearing elements 300A, 300B, 310A, 310B are received in receptacles 301A, 301B, 311A, 311B on the respective body 303, 313 of the associated guiding section 30, 31. The bearing elements 300A, 300B, 310A, 310B are rotationally fixed with respect to the body 303, 313. For this, fixation elements 302A, 302B, 312A, 312B in the shape of clamping screws in openings 304A, 304B, 314A, 314B of the body 303, 313 are brought into engagement with the respective bearing element 300A, 300B, 310A, 310B to clamp the bearing element 300A, 300B, 310A, 310B within the associated receptacle 301A, 301B, 311A, 311B.

The guiding device 3 hence acts as a gliding bearing for the tube 5 and does not comprise any rotational parts.

As visible for example from Figs. 4 and 5, the bearing elements 300A, 300B, 310A, 310B in the shape of the spherical balls in the shown embodiment are arranged in a common plane, in that the bearing elements 300A, 300B, 310A, 310B are axially arranged at the same position with respect to the longitudinal direction X (the common plane extends through the centers of all bearing elements 300A, 300B, 310A, 310B).

Herein, each guiding section 30, 31 comprises one pair of bearing elements 300A, 300B, 310A, 310B, the bearing elements 300A, 300B, 310A, 310B being arranged such that each bearing element 300A, 300B, 310A, 310B is in abutment with the tube 5 when the tube 5 is received in between the guiding sections 30, 31.

Due to their spherical shape, the bearing elements 300A, 300B, 310A, 310B comprise a convex curvature in a plane spanned by the longitudinal direction X and a transverse, radial direction pointing towards and through the respective bearing element 300A, 300B, 310A, 310B. Because of the convex curvature, each bearing element 300A, 300B, 310A, 310B is in abutment with the tube 5 only at a spatially confined, spot-like location.

Ideally each bearing element 300A, 300B, 310A, 310B is in abutment with the tube 5 at a point-like location. Due to interaction of the respective bearing element 300A, 300B, 310A, 310B with the tube 5, however, the tube 5 is supported at a finite contact area with respect to the respective bearing element 300A, 300B, 310A, 310B.

As schematically shown in Fig. 3, the guiding sections 30, 31 are tensioned with respect to one another by means of a tensioning device 32, for example in the shape of a mechanical spring or a hydraulic or pneumatic tensioning device. The tensioning device 32 may for example act onto the guiding section 31, wherein the other guiding section 30 is held stationary. By means of the tensioning device 32 a contact pressure is established such that the bearing elements 300A, 300B, 310A, 310B abut the tube 5 with a defined contact force for guiding the tube 5 at the guiding device 3.

In the shown embodiment, the bearing elements 300A, 300B, 310A, 310B in the shape of the spherical balls are for example made from a material which is harder than the material of the tube 5. The bearing elements 300A, 300B, 310A, 310B may for example be made from a rolling bearing steel material, a ceramics material or a hard metal material. The bearing elements 300A, 300B, 310A, 310B in particular may comprise a hardness in the range between 55 HRC to 70 HRC (in particular for a rolling bearing steel material).

Because the hardness of the bearing elements 300A, 300B, 310A, 310B is larger than the hardness of the tube 5, friction in between the bearing elements 300A, 300B, 310A, 310B and the tube 5 is predominantly governed by the bearing elements 300A, 300B, 310A, 310B. By using bearing elements 300A, 300B, 310A, 310B having a small surface roughness, friction in between the bearing elements 300A, 300B, 310A, 310B and the tube 5 may be reduced, such that the tube 5 may be guided on the guiding device 3 with minimal friction.

Because the bearing elements 300A, 300B, 310A, 310B are made from a hard material, in addition a wear and tear and a deformation over the lifespan of the guiding device 3 may be reduced, such that the lifespan of the guiding device 3 may be increased.

The bearing elements 300A, 300B, 310A, 310B may be standard, commercially available bearing balls, allowing to reduce costs for the guiding device 3 and hence for the overall production system 1.

Because the bearing elements 300A, 300B, 310A, 310B are arranged in a common plane, the length of the guiding device 3 (measured along the longitudinal direction X) may be reduced. This allows to reduce a length of the tube 5 which cannot the use to produce medical devices 50, in that the length of the tube 5 to be received in the guiding device 3 is small.

Because the bearing elements 300A, 300B, 310A, 310B are arranged in a common plane and comprise a convex curvature in a plane spanned by the longitudinal direction X and a transverse, radial direction pointing through the respective bearing element 300A, 300B, 310A, 310B, the guiding device 3 predominantly provides a support along a vertical direction and along a transverse, horizontal direction, hence in a plane perpendicular to the longitudinal direction X. The guiding device 3 thus, in the shown embodiment, provides only for a limited angular support. This may facilitate the use of the production system 1, in that it is not required to align the guiding device 3 in an angular direction with respect to the positioning device 2, which may save time and complexity of using the production system 1.

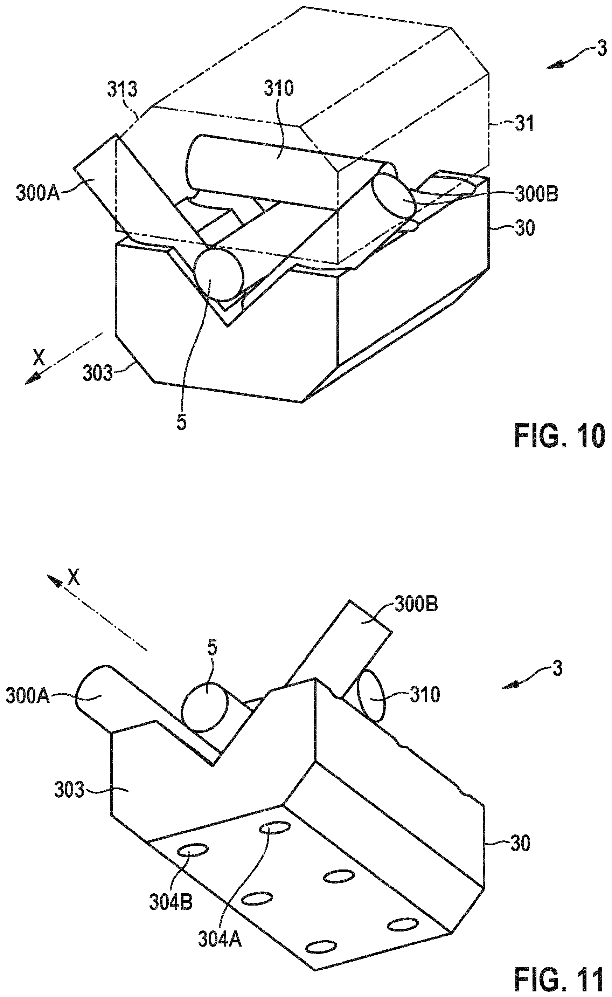

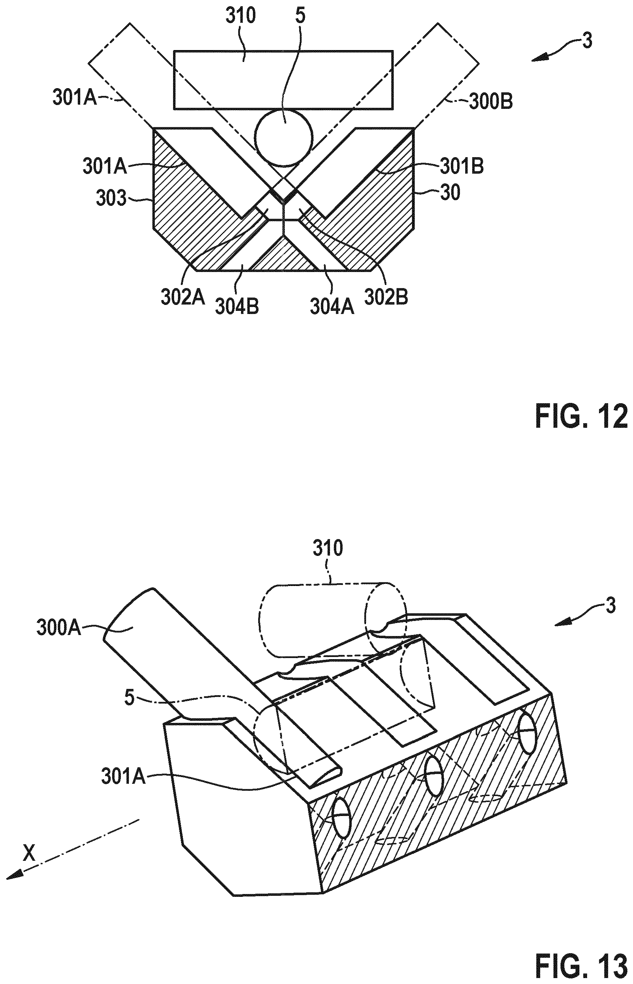

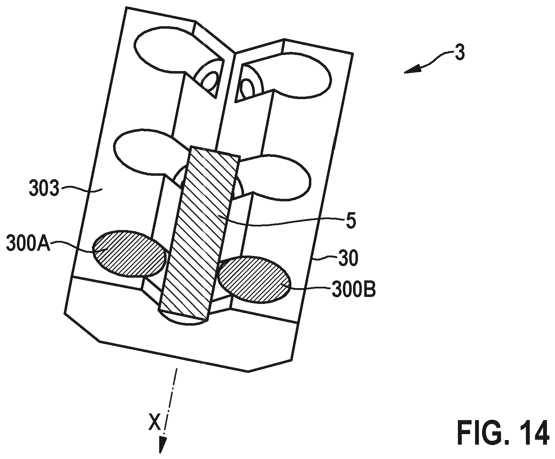

Referring now to Figs. 10 to 14, in another embodiment of a guiding device 3 bearing elements 300A, 300B, 310 are formed by cylindrical pins, wherein a pair of bearing elements 300A, 300B is fixedly arranged on the body 303 of a first guiding section, and a single bearing element 310 is arranged on the body 313 of the second guiding section.

The guiding sections 30, 31 may be elastically tensioned with respect to one another using a tensioning device 32, as schematically shown in Fig. 3.

In the embodiment of Figs. 10 to 14, the bearing elements 300A, 300B, 310 in the shape of the cylindrical pins each extend along an associated longitudinal axis of extension L1, L2, L3, wherein the axes of extension L1, L2, L3 are oriented perpendicularly to the longitudinal direction X.

The axes of extension L1, L2 of the bearing elements 300A, 300B of the guiding section 30 herein are arranged in a common plane, whereas the axis of extension L3 of the bearing element 310 of the guiding section 31 is arranged in a plane which is offset with respect to the plane of the bearing elements 300A, 300B of the guiding section 30.

The bearing elements 300A, 300B of the guiding section 30 hence provide support from below, whereas the bearing element 310 of the guiding section 31 provides support from above at an axial location which is offset to the axial location of the bearing elements 300A, 300B.

Due to the cylindrical shape of the bearing elements 300A, 300B, 310, each bearing element 300A, 300B, 310 comprises a convex curvature in a plane spanned by the longitudinal direction X and a transverse direction perpendicular to the longitudinal direction X and the respective axis of extension L1, L2, L3 of the respective bearing element 300A, 300B, 310. Due to the convex curvature, each bearing element 300A, 300B, 310 contacts the tube 5, when received between the guiding sections 30, 31, at a spatially confined, spot-like location, such that a contact area in between the tube 5 and the bearing elements 300A, 300B, 310 is reduced.

As described above for the embodiment of Figs. 2 to 9, the bearing elements 300A, 300B, 310 beneficially are made from a material which comprises a hardness larger than the hardness of the material of the tube 5. The bearing elements 300A, 300B, 310 in particular may be made from a rolling bearing steel material, a ceramics material or a hard metal material. The bearing elements 300A, 300B, 310 may be commercially available as standard bearing elements.

The bearing elements 300A, 300B, 310 in the embodiment of Figs. 10 to 14 are each received in an associated receptacle 301A, 301B of the guiding sections 30, 31, wherein the bearing elements 300A, 300B, 310 are rotationally fixed to the respective body 303, 313 of the guiding section 30, 31.

It is to be noted that any combination of bearing elements 300A, 300B, 310, 310A, 310B of the embodiments of Figs. 2 to 9 and Figs. 10 to 14 may be used. For example, a combination of spherical balls and cylindrical pins may be employed. For example, a first guiding section 30 may comprise an arrangement of one or multiple bearing elements in the shape of spherical balls, whereas a second guiding section 31 may comprise an arrangement of one or multiple bearing elements in the shape of cylindrical pins.

It further shall be noted that the number of bearing elements 300A, 300B, 310, 310A, 310B may differ from the number employed in the embodiments of Figs. 2 to 9 and 10 to 14. For example, only two bearing elements may be used. In other embodiments, more than four bearing elements may be used.

The idea of the invention is not limited to the embodiments described above, but may be implemented in an entirely different fashion.

The production system in particular can be used to produce medical devices in the shape of stents or scaffolds from a cylindrical tube using a laser cutting device. A production system of the kind described above may, in principle, also be used to produce other medical devices from a tube, for example made from a nitinol material, a stainless steel material, or a bioresorbable material such as a magnesium material.

### List of reference numerals

- 1: Production system
- 2: Positioning device
- 3: Guiding device
- 30: First guiding section
- 300A, B: Bearing element
- 301A, B: Receptacle
- 302A, B: Fixation element
- 303: Body
- 304A, B: Opening
- 31: Second guiding section
- 310: Bearing element
- 310A, B: Bearing element
- 311A, B: Receptacle
- 312A, B: Fixation element
- 313: Body
- 314A, B: Opening
- 32: Tensioning device
- 4: Laser device
- 5: Tube
- 50: Medical device (tubular shaped semi-finished product, e.g. stent or scaffold)
- B: Laser beam
- F: Guiding pressure
- L1-L3: Axis of extension
- X: Longitudinal direction
- Y: Rotational direction

## Claims

1. A production system (1) for producing a medical device (50) from a tube (5), comprising:
a positioning device (2) for positioning the tube (5), the positioning device (2) being configured to hold the tube (5) such that a portion of the tube (5) extends from the positioning device (2) along a longitudinal direction (L);
a guiding device (3) for guiding said portion of the tube (5);
a laser cutting device (4) for forming said medical device (50) from said portion of the tube (5);
wherein said guiding device (3) comprises a multiplicity of bearing elements (300A, 300B, 310, 310A, 310B) each configured to abut said portion of the tube (5) and each having a convex curvature in a plane spanned by the longitudinal direction (X) and a transverse direction perpendicular to the longitudinal direction (X);
**characterized in that** the guiding device (3) further comprises a first guiding section (30) having a first body (303) and a second guiding section (31) having a second body (313), wherein a first subgroup of the multiplicity of bearing elements (300A, 300B, 310, 310A, 310B) is arranged on the first body (303) of the first guiding section (30) and a second subgroup of the multiplicity of bearing elements (300A, 300B, 310, 310A, 310B) is arranged on the second body (313) of the second guiding section (31), wherein the first subgroup of the multiplicity of bearing elements (300A, 300B, 310, 310A, 310B) is rotationally fixed to the first body (303) of the first guiding section (30), and/or the second subgroup of the multiplicity of bearing elements (300A, 300B, 310, 310A, 310B) is rotationally fixed to the second body (313) of the second guiding section (31).

2. The production system (1) according to claim 1, **characterized in that** the bearing elements (300A, 300B, 310, 310A, 310B) are made from a material comprising a hardness larger than the hardness of the tube (5).

3. The production system (1) according to claim 1 or 2, **characterized in that** the bearing elements (300A, 300B, 310, 310A, 310B) are made from a rolling bearing steel material, a ceramics material or a hard metal material.

4. The production system (1) according to one of claims 1 to 3, **characterized in that** at least one of the bearing elements (300A, 300B, 310, 310A, 310B) is formed by a spherical ball.

5. The production system (1) according to one of the preceding claims, **characterized in that** at least one of the bearing elements (300A, 300B, 310, 310A, 310B) is formed by a cylindrical pin.

6. The production system (1) according to claim 5, **characterized in that** the at least one of the bearing elements (300A, 300B, 310, 310A, 310B) formed by a cylindrical pin extends longitudinally along an axis of extension (L1-L3), wherein the axis of extension (L1-L3) lies in a plane oriented perpendicularly to the longitudinal direction (X).

7. The production system (1) according to one of the claims 1 to 6, **characterized in that** the bearing elements (300A, 300B, 310, 310A, 310B) are arranged in a common plane oriented perpendicularly to the longitudinal direction (X).

8. The production system (1) according to one of claims 1 to 6, **characterized in that** at least one of the bearing elements (300A, 300B, 310, 310A, 310B) is arranged in a first plane oriented perpendicularly to the longitudinal direction (X), and at least one other of the bearing elements (300A, 300B, 310, 310A, 310B) is arranged in a second plane oriented perpendicularly to the longitudinal direction (X) and displaced with respect to said first plane along the longitudinal direction (X).

9. The production system (1) according to claim 1, **characterized in that** the first guiding section (30) and the second guiding section (31) are configured to receive said portion of the tube (5) between the first guiding section (30) and the second guiding section (31).

10. The production system (1) according to claim 9, **characterized in that** the first guiding section (30) and the second guiding section (31) are tensioned with respect to one another to apply a guiding pressure (F) to the portion of the tube (5) received between the first guiding section (30) and the second guiding section (31).

11. The production system (1) according to one of claims 1, 9 or 10, **characterized in that** the first body (303) comprises at least one first receptacle (301A, 301B) in which one of the bearing elements of said first subgroup is received, and/or the second body (313) comprises at least one second receptacle (311A, 311B) in which one of the bearing elements of said second subgroup is received.

12. The production system (1) according to claim 11, **characterized in that** said one of the bearing elements of said first subgroup is clamped in said at least one first receptacle (301A, 301B) such that said one of the bearing elements of said first subgroup is rotationally fixed in said at least one first receptacle (301A, 301B), and/or said one of the bearing elements of said second subgroup is clamped in said at least one second receptacle (311A, 311B) such that said one of the bearing elements of said second subgroup is rotationally fixed in said at least one second receptacle (311A, 311B).

13. The production system (1) according to one of the preceding claims, **characterized in that** the laser cutting device (4) is configured to form said medical device (50) on an end of said portion of the tube (5), the guiding device (3) being arranged, when viewed along the longitudinal direction (X), in between the positioning device (2) and the laser cutting device (4).

## Patentansprüche

1. Ein Produktionssystem (1) zur Herstellung eines Medizinprodukts (50) aus einem Rohr (5), umfassend:
eine Positioniervorrichtung (2) zum Positionieren des Rohrs (5), wobei die Positioniervorrichtung (2) dazu ausgebildet ist, das Rohr (5) derart zu halten, dass ein Abschnitt des Rohrs (5) aus der Positioniervorrichtung (2) entlang einer Längsrichtung (L) herausragt;
eine Führungsvorrichtung (3) zum Führen des Abschnitts des Rohrs (5); eine Laserschneidvorrichtung (4) zum Formen des Medizinprodukts (50) aus dem Abschnitt des Rohrs (5);
wobei die Führungsvorrichtung (3) eine Vielzahl von Lagerelementen (300A, 300B, 310, 310A, 310B) umfasst, die jeweils dazu ausgebildet sind, an dem Abschnitt des Rohrs (5) anzuliegen, und die jeweils eine konvexe Krümmung in einer durch die Längsrichtung (X) und eine zur Längsrichtung (X) senkrechte Querrichtung aufgespannten Ebene aufweisen;
**dadurch gekennzeichnet, dass** die Führungsvorrichtung (3) ferner einen ersten Führungsabschnitt (30) mit einem ersten Körper (303) und einen zweiten Führungsabschnitt (31) mit einem zweiten Körper (313) umfasst, wobei eine erste Untergruppe der Vielzahl von Lagerelementen (300A, 300B, 310, 310A, 310B) an dem ersten Körper (303) des ersten Führungsabschnitts (30) angeordnet ist und eine zweite Untergruppe der Vielzahl von Lagerelementen (300A, 300B, 310, 310A, 310B) an dem zweiten Körper (313) des zweiten Führungsabschnitts (31) angeordnet ist, wobei die erste Untergruppe der Vielzahl von Lagerelementen (300A, 300B, 310, 310A, 310B) drehfest mit dem ersten Körper (303) des ersten Führungsabschnitts (30) verbunden ist, und/oder die zweite Untergruppe der Vielzahl von Lagerelementen (300A, 300B, 310, 310A, 310B) drehfest mit dem zweiten Körper (313) des zweiten Führungsabschnitts (31) verbunden ist.

2. Das Produktionssystem (1) nach Anspruch 1, **dadurch gekennzeichnet, dass** die Lagerelemente (300A, 300B, 310, 310A, 310B) aus einem Material hergestellt sind, das eine Härte aufweist, die größer ist als die Härte des Rohrs (5).

3. Das Produktionssystem (1) nach Anspruch 1 oder 2, **dadurch gekennzeichnet, dass** die Lagerelemente (300A, 300B, 310, 310A, 310B) aus einem Wälzlagerstahlmaterial, einem Keramikmaterial oder einem Hartmetallmaterial hergestellt sind.

4. Das Produktionssystem (1) nach einem der Ansprüche 1 bis 3, **dadurch gekennzeichnet, dass** wenigstens eines der Lagerelemente (300A, 300B, 310, 310A, 310B) durch eine kugelförmige Kugel gebildet ist.

5. Das Produktionssystem (1) nach einem der vorangehenden Ansprüche, **dadurch gekennzeichnet, dass** wenigstens eines der Lagerelemente (300A, 300B, 310, 310A, 310B) durch einen zylindrischen Stift gebildet ist.

6. Das Produktionssystem (1) nach Anspruch 5, **dadurch gekennzeichnet, dass** das wenigstens eine der Lagerelemente (300A, 300B, 310, 310A, 310B), das durch einen zylindrischen Stift gebildet ist, sich longitudinal entlang einer Ausdehnungsachse (L1-L3) erstreckt, wobei die Ausdehnungsachse (L1-L3) in einer Ebene liegt, die senkrecht zur Längsrichtung (X) ausgerichtet ist.

7. Das Produktionssystem (1) nach einem der Ansprüche 1 bis 6, **dadurch gekennzeichnet, dass** die Lagerelemente (300A, 300B, 310, 310A, 310B) in einer gemeinsamen Ebene angeordnet sind, die senkrecht zur Längsrichtung (X) ausgerichtet ist.

8. Das Produktionssystem (1) nach einem der Ansprüche 1 bis 6, **dadurch gekennzeichnet, dass** wenigstens eines der Lagerelemente (300A, 300B, 310, 310A, 310B) in einer ersten Ebene angeordnet ist, die senkrecht zur Längsrichtung (X) ausgerichtet ist, und wenigstens ein weiteres der Lagerelemente (300A, 300B, 310, 310A, 310B) in einer zweiten Ebene angeordnet ist, die senkrecht zur Längsrichtung (X) ausgerichtet ist und gegenüber der ersten Ebene entlang der Längsrichtung (X) versetzt ist.

9. Das Produktionssystem (1) nach Anspruch 1, **dadurch gekennzeichnet, dass** der erste Führungsabschnitt (30) und der zweite Führungsabschnitt (31) dazu ausgebildet sind, den Abschnitt des Rohrs (5) zwischen dem ersten Führungsabschnitt (30) und dem zweiten Führungsabschnitt (31) aufzunehmen.

10. Das Produktionssystem (1) nach Anspruch 9, **dadurch gekennzeichnet, dass** der erste Führungsabschnitt (30) und der zweite Führungsabschnitt (31) gegeneinander vorgespannt sind, um einen Führungsdruck (F) auf den Abschnitt des Rohrs (5) auszuüben, der zwischen dem ersten Führungsabschnitt (30) und dem zweiten Führungsabschnitt (31) aufgenommen ist.

11. Das Produktionssystem (1) nach einem der Ansprüche 1, 9 oder 10, **dadurch gekennzeichnet, dass** der erste Körper (303) wenigstens eine erste Aufnahme (301A, 301B) umfasst, in der eines der Lagerelemente der ersten Untergruppe aufgenommen ist, und/oder der zweite Körper (313) wenigstens eine zweite Aufnahme (311A, 311B) umfasst, in der eines der Lagerelemente der zweiten Untergruppe aufgenommen ist.

12. Das Produktionssystem (1) nach Anspruch 11, **dadurch gekennzeichnet, dass** das eine der Lagerelemente der ersten Untergruppe in der wenigstens einen ersten Aufnahme (301A, 301B) derart geklemmt ist, dass das eine der Lagerelemente der ersten Untergruppe in der wenigstens einen ersten Aufnahme (301A, 301B) drehfest ist, und/oder das eine der Lagerelemente der zweiten Untergruppe in der wenigstens einen zweiten Aufnahme (311A, 311B) derart geklemmt ist, dass das eine der Lagerelemente der zweiten Untergruppe in der wenigstens einen zweiten Aufnahme (311A, 311B) drehfest ist.

13. Das Produktionssystem (1) nach einem der vorangehenden Ansprüche, **dadurch gekennzeichnet, dass** die Laserschneidvorrichtung (4) dazu ausgebildet ist, das Medizinprodukt (50) an einem Ende des Abschnitts des Rohrs (5) zu formen, wobei die Führungsvorrichtung (3), bei Betrachtung entlang der Längsrichtung (X), zwischen der Positioniervorrichtung (2) und der Laserschneidvorrichtung (4) angeordnet ist.

## Revendications

1. Système de production (1) destiné à la production d'un dispositif médical (50) à partir d'un tube (5), comprenant :
un dispositif de positionnement (2) destiné au positionnement du tube (5), le dispositif de positionnement (2) étant configuré pour retenir le tube (5) de telle sorte qu'une partie du tube (5) s'étend à partir du dispositif de positionnement (2) le long d'une direction longitudinale (L) ;
un dispositif de guidage (3) destiné au guidage de ladite portion du tube (5) ;
un dispositif de découpe au laser (4) destiné au formage dudit dispositif médical (50) à partir de ladite partie du tube (5) ;
dans lequel ledit dispositif de guidage (3) comprend une multiplicité d'éléments d'appui (300A, 300B, 310, 310A, 310B) configurés chacun pour venir en butée contre ladite partie du tube (5) et ayant chacun une courbure convexe dans un plan couvert par la direction longitudinale (X) et une direction transversale perpendiculaire à la direction longitudinale (X) ;
**caractérisé en ce que** le dispositif de guidage (3) comprend en outre une première section de guidage (30) présentant un premier corps (303) et une seconde section de guidage (31) présentant un second corps (313), un premier sous-groupe de la multiplicité d'éléments d'appui (300A, 300B, 310, 310A, 310B) étant agencé sur le premier corps (303) de la première section de guidage (30) et un second sous-groupe de la multiplicité d'éléments d'appui (300A, 300B, 310, 310A, 310B) étant agencé sur le second corps (313) de la seconde section de guidage (31), le premier sous-groupe de la multiplicité d'éléments d'appui (300A, 300B, 310, 310A, 310B) étant fixé au premier corps (303) de la première section de guidage (30) de façon à pouvoir tourner, et/ou le second sous-groupe de la multiplicité d'éléments d'appui (300A, 300B, 310, 310A, 310B) étant fixé au second corps (313) de la seconde section de guidage (31) de façon à pouvoir tourner.

2. Système de production (1) selon la revendication 1, **caractérisé en ce que** les éléments d'appui (300A, 300B, 310, 310A, 310B) sont constitués d'un matériau présentant une dureté supérieure à la dureté du tube (5).

3. Système de production (1) selon la revendication 1 ou 2, **caractérisé en ce que** les éléments d'appui (300A, 300B, 310, 310A, 310B) sont constitués d'un matériau d'acier de palier à roulement, d'un matériau céramique ou d'un matériau métallique dur.

4. Système de production (1) selon l'une des revendications 1 à 3, **caractérisé en ce qu'**au moins l'un des éléments d'appui (300A, 300B, 310, 310A, 310B) est formé par une bille sphérique.

5. Système de production (1) selon l'une des revendications précédentes, **caractérisé en ce qu'**au moins l'un des éléments d'appui (300A, 300B, 310, 310A, 310B) est formé par une goupille cylindrique.

6. Système de production (1) selon la revendication 5, **caractérisé en ce que** l'élément d'appui, au moins au nombre de un, parmi les éléments d'appuis (300A, 300B, 310, 310A, 310B) formé par une goupille cylindrique s'étend longitudinalement le long d'un axe d'extension (L1-L3), l'axe d'extension (L1-L3) reposant dans un plan orienté perpendiculairement à la direction longitudinale (X).

7. Système de production (1) selon l'une des revendications 1 à 6, **caractérisé en ce que** les éléments d'appui (300A, 300B, 310, 310A, 310B) sont agencés dans un plan commun orienté perpendiculairement à la direction longitudinale (X).

8. Système de production (1) selon l'une des revendications 1 à 6, **caractérisé en ce qu'**au moins l'un des éléments d'appui (300A, 300B, 310, 310A, 310B) est agencé dans un premier plan orienté perpendiculairement à la direction longitudinale (X), et au moins un autre des éléments d'appui (300A, 300B, 310, 310A, 310B) est agencé dans un second plan orienté perpendiculairement à la direction longitudinale (X) et déplacé par rapport audit premier plan le long de la direction longitudinale (X).

9. Système de production (1) selon la revendication 1, **caractérisé en ce que** la première section de guidage (30) et la seconde section de guidage (31) sont configurées pour recevoir ladite partie du tube (5) entre la première section de guidage (30) et la seconde section de guidage (31).

10. Système de production (1) selon la revendication 9, **caractérisé en ce que** la première section de guidage (30) et la seconde section de guidage (31) sont mises en tension l'une par rapport à l'autre pour appliquer une pression de guidage (F) sur la partie du tube (5) reçue entre la première section de guidage (30) et la seconde section de guidage (31).

11. Système de production (1) selon l'une des revendications 1, 9 ou 10, **caractérisé en ce que** le premier corps (303) comprend au moins un premier réceptacle (301A, 301B) dans lequel l'un des éléments d'appui dudit premier sous-groupe est reçu, et/ou le second corps (313) comprend au moins un second réceptacle (311A, 311B) dans lequel l'un des éléments d'appui dudit second sous-groupe est reçu.

12. Système de production (1) selon la revendication 11, **caractérisé en ce que** ledit élément d'appui parmi les éléments d'appui dudit premier sous-groupe est serré dans ledit premier réceptacle (301A, 301B), au moins au nombre de un, de telle sorte que ledit élément d'appui parmi les éléments d'appui dudit premier sous-groupe est fixé dans ledit premier réceptacle (301A, 301B), au moins au nombre de un, de façon à pouvoir tourner, et/ou ledit élément d'appui parmi les éléments d'appui dudit second sous-groupe est serré dans ledit second réceptacle (311A, 311B), au moins au nombre de un, de telle sorte que ledit élément parmi les éléments d'appui dudit second sous-groupe est fixé dans ledit second réceptacle (311A, 311B), au moins au nombre de un, de façon à pouvoir tourner.

13. Système de production (1) selon l'une des revendications précédentes, **caractérisé en ce que** le dispositif de découpe au laser (4) est configuré pour former ledit dispositif médical (50) sur une extrémité de ladite partie du tube (5), le dispositif de guidage (3) étant agencé, lorsqu'il est vu le long de la direction longitudinale (X), entre le dispositif de positionnement (2) et le dispositif de découpe au laser (4).
